# EUROPEAN PATENT APPLICATION

(11) **EP 2 175 023 A1**
(43) Date of publication of application: **14.04.2010**
(21) Application number: 09075334.4
(22) Date of filing: 24.07.2009
(51) Int. Cl.: C12N 15/11

(54) **Anti-tumor activity of small double-stranded oligodeoxynucleotides targeting telomerase RNA**

(30) Priority: 31.07.2008 EP 08075678
(71) Applicant: The University of Zurich, 8006 Zürich (CH)
(72) Inventor: Mölling, Karin Prof. Dr., 14195 Berlin (DE)
(74) Representative: Boeckh, Tobias

(57) **Abstract**

The invention refers to an antitumor and/or anti-metastatic therapeutic, comprising siDNA oligonucleotides that bind to one or more RNA target regions of the RNA component of telomerase. The siDNA oligonucleotides comprise of one strand which is fully complementary to the template region of the RNA component of Telomerase and a second strand partially complementary to the antisense strand. The siDNA oligos bind the Telomerase RNA and lead to its inactivation.

## Description

### FIELD OF THE INVENTION

The invention refers to an antitumor and/or anti-metastatic therapeutic, comprising siDNA oligonucleotides that bind to one or more RNA target regions of the RNA component of telomerase. Telomerase is up-regulated in cancer cells and is therefore a candidate target for anticancer drugs. The destruction of telomerase RNA and inactivation of telomerase is achieved by partially double-stranded, hairpin-loop-structured oligodeoxynucleotides (siDNA). These oligonucleotides bind the Telomerase RNA and lead to RNA cleavage by RNase H. siDNA oligonucleotides comprise of one strand which is fully complementary to the template region of the RNA component of Telomerase and a second strand partially complementary to the antisense strand, which protects against nucleases. Both strands are connected by a linker of four thymidines. The siDNA is superior to siRNA because the formation of RNA-DNA hybrids is preferred over double-stranded DNA or double-stranded RNA, which forms as a tertiary structure in RNA genomes, and siDNA is easier to synthesize and is more stable than siRNA. It can also be combined with other siDNAs targeted against various target regions of the RNA component of telomerase.

### BACKGROUND OF THE INVENTION

Telomeres are specialized DNA-protein structures located at the ends of chromosomes. Human telomeric DNA, usually 5-20 kb in length, consists of repetitive 5'-TTAGGG-3' sequences. Telomeres serve to protect chromosomes against rearrangement, end-to-end fusion, degradation and chromosomal loss, thus contributing to chromosomal stabilization. In human somatic cells, due to the inability of DNA polymerase to completely replicate the end of chromosomes, telomeres are shortened between 50-200 base pairs with each cell division.

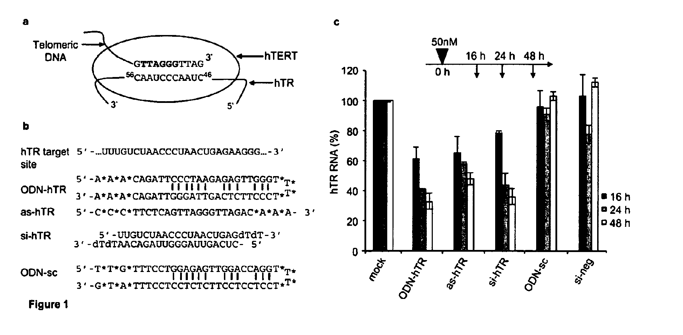

This progressive telomere shortening eventually reaches a critical stage leading to telomere dysfunction and senescence. Reversion of this degradation by telomere maintenance mechanism increases cellular replicative capacity. A telomere maintenance mechanism is provided by a cellular reverse transcriptase (RT) enzyme complex called telomerase. First identified in ciliated protozoa, telomerase is known to be almost universally conserved in eukaryotes. It is an obligate ribonucleoprotein whose catalytic function depends minimally on two components: the TERT (telomerase reverse transcriptase, or hTERT in humans) protein and TR (telomerase RNA, or hTR in humans). The hTR contains a short segment, which encodes the cognate telomere repeat (as demonstrated in Figure 1a), and this segment serves as the template for reverse transcription by hTERT (Nakamura et al., Science 277: 955-959, 1997). Although sharing many properties of conventional RTs, hTERT also exhibits unique features; the primary ones are its utilization of a template embedded in the hTR and its ability to add multiple components of the template through repeated cycles of extension and translocation reactions. This ability of telomerase is predicated on telomerase-specific structures located both within and outside of the RT domain.

Telomerase is strongly repressed in normal somatic tissues but expressed in highly proliferative ones, including ovaries, testis, and hematopoietic tissues. Remarkably, telomerase is also strongly upregulated in most cancer cells; a feature that accounts for their proliferative capacity (Blackburn, Mol Cancer Res 9: 477-482, 2005). In fact, the ectopic expression of hTERT in combination with two oncogenes (the simian virus 40 large-T oncoprotein and an oncogenic allele of H-ras) results in tumorigenic conversion of normal human epithelial and fibroblast cells (Hahn et al., Nat Med 5: 1164-1170, 1999). Based on these findings, it is not surprising that telomerase has attracted considerable attention as a plausible target for cancer therapy. First report of successful inhibition of telomerase activity involved the use of a single-stranded antisense oligonucleotide against the hTR (Feng et al., Science 269: 1236-1241, 1995). Later, others have used different expression vectors and modified oligonucleotides against the hTR and hTERT (Bisoffi et al., Eur J Cancer 34: 1242-1249, 1998; Zhang et al., World J Gastroenterol 6: 430-432, 2000; Braasch and Corey, Biochemistry 41: 4503-4510, 2002; Zhang et al., Oncogene 22: 2405-2416, 2003).

There are several ways to inactivate RNAs inside the cell. One of these is the well-known antisense strategy, whereby a single stranded DNA oligonucleotide is directed to a target RNA and hybridizes by Watson-Crick based pairing. The RNA-DNA hybrid is recognized by an enzyme designated as ribonuclease H or RNA H, whereby H indicates the RNA-DNA hybrid substrates. It cleaves the RNA within the hybrid region.

A second mechanism is based on a triple helix, whereby the target strand may be single or double stranded DNA and the third strand binds to this double stranded structure. Again one strand hybridizes to the target strand by Watson-Crick base pairing, whereas the third strand binds to Hoogsteen base-pairing. A triple helix is characterized by its resistance to cleavage by cellular enzymes and leads to inhibition of expression by unknown mechanisms, possibly translation arrest. The role of a triple helix inside the cell is unclear.

A third mechanism is based on inactivating a target RNA by double stranded RNA, the so-called 'short interfering' siRNA. Here one strand of the siRNA is poly-hybridized to the target RNA by Watson Crick base pairing, the so-called antisense strand. A second RNA strand, the passenger strand, is fully complementary to the antisense strand and only transiently required for inactivation of the RNA. It is removed in a so-called RISC-complex. This mechanism of inactivation of the target RNA by siRNA is called silencing. The enzyme required for RNA silencing is designated as Argonaute. The discovery of this mechanism was awarded with the Nobel Prize in 2006. In all cases gene inactivation is achieved by small oligonucleotides about 20 nucleotides in length, which may or may not contain a linker region between the two nucleic acid strands.

A fourth mechanism is the use of short interfering DNA (siDNA) to inactivate viral RNA genomes. As shown by the inventor, this mechanism activates the RNase H of the virus and leads to silencing of the viral RNA (Matzen et al, Nature Biotechnol. 25, 669-674, 2007). The RNase H is a Hybrid-specific RNase which was discovered by Moelling et al (Nature New Biology 234, 240-243, 1971). siDNA oligonucleotides have recently been applied in silencing various RNA targets. The oligonucleotides contain hairpin-loop structures, whereby one strand is fully complementary to the target RNA and the other one is selected using Hoogsteen rules. The oligodeoxynucleotide A (ODN A) has been successfully targeted to the highly conserved polypurine tract (PPT) required for the second strand DNA synthesis of HIV-1 (Moelling et al., FEBS Lett 580: 3454-3450, 2006). The ODN A prematurely activates the HIV-1 RNase H which inactivates the viral RNA in cell free HIV particles. This inhibitory effect of ODN A was highly sequence-specific and was more effective than the antisense strand alone.

Success in the targeting of viral RNA using siDNA has been demonstrated, but no prior art exists disclosing the use of siDNA to target non-viral RNAs. The present invention demonstrates a novel and unexpected success in using siDNA oligonucleotides, capable of binding to one or more RNA target regions of the RNA component of telomerase, to inactivate Telomerase RNA and serve as an antitumor and/or anti-metastatic therapeutic for inhibiting tumor growth and/or metastasis.

### FIGURES AND LEGENDS

Figure 1. Oligonucleotides targeting the human telomerase RNA (hTR).
Figure 2. Oligonucleotides targeting murine telomerase RNA (mTR).
Figure 3. In vitro analysis of tumor cell proliferation.
Figure 4. Mechanism of ODN-mediated hTR downregulation.
Figure 5. In vivo anti-metastatic effect.

### SUMMARY OF THE INVENTION

One problem to be solved by the present invention is to provide an effective antitumor and/or anti-metastatic therapeutic for inhibiting tumor growth and/or metastasis using siDNA. This problem is solved by the features of the independent claims.

An object of the invention is therefore to provide isolated and purified siDNA oligonucleotides for binding to one or more RNA target regions of the RNA component of telomerase, comprising a partially double-stranded, hairpin-loop structure with an antisense-strand complementary to one or more of said RNA target regions and a second strand, partially complementary to the antisense strand, wherein the siDNA oligonucleotides bind to one or more target regions of the RNA component of telomerase and serve as an antitumor and/or anti-metastatic therapeutic for inhibiting tumor growth and/or metastasis.

Another object of the invention is therefore to provide a method for the treatment of cancer and/or tumor growth comprising administering siDNA, or combination of two or three siDNAs according to the present invention, wherein the siDNA oligonucleotides bind to one or more target regions of the RNA component of telomerase and serve as an antitumor and/or anti-metastatic therapeutic for inhibiting tumor growth and/or metastasis.

Another object of the invention is therefore to provide a pharmaceutical composition as an antitumor and/or anti-metastatic therapeutic for inhibiting tumor growth and/or metastasis comprising siDNA oligonucleotides according to the present invention, wherein the siDNA oligonucleotides are capable of binding to one or more target regions of the RNA component of telomerase. Such a pharmaceutical composition according to the present invention is also intended to contain any additional components necessary for the administration or delivery of the composition during treatment. Such a pharmaceutical composition is intended for the treatment of all cancers, and in particular lung cancers such as malignant lung metastases.

A further object of the invention is therefore to provide a method for monitoring the RNA component of telomerase after treatment with siDNA oligonucleotides according to the present invention, comprising DNA oligonucleotides for the detection of the target RNA in order to determine the effect of the siDNA-mediated silencing.

An additional unique feature of the present invention is that the siDNA oligonucleotides may be complementary to the template region of the RNA component of telomerase of humans.

The siDNA oligonucleotides of the present invention are claimed as antitumor and/or anti-metastatic therapeutics, wherein the siDNA molecule is complementary to one or more conserved regions of the RNA component of telomerase, of at least 20 nucleotides in length, wherein the antisense strand of siDNA is more than 80%, more preferably more than 90% homologous to the target telomerase RNA. Further, the second strand is connected to the antisense strand through a thymidine linker, preferred 4 nucleotides in length, where the second strand has a homology of 40 to 60% to the antisense-strand and is thus partially complementary to the antisense-strand.

This invention provides surprising and unexpected success as an anti-cancer therapeutic by inactivating the RNA component of telomerase as demonstrated in the examples below. siDNA oligonucleotides (ODNs) according to the present invention were designed to target the hTR in the human cervical carcinoma cell line (Hela cells) and mouse telomerase RNA (mTR) in the murine metastatic melanoma cell line (B16-F10 cells), respectively. The examples contained within indicate that siDNA ODNs are able to inhibit the hTR by 68% and the mTR by 81% in the respective cell lines. This correlated with ODN-mediated inhibition of cell proliferation and induction of apoptosis. The inhibition of the hTR was enhanced by overexpression of the p66 subunit of HIV-1 reverse transcriptase/ribonuclease H (RT/RNase H) and decreased by knocking down of the cellular RNases H suggesting their contribution. Furthermore, a 70% reduction in numbers of metastases was observed after intravenous administration of ODN-transfected B16-F10 cells in C57BL/6 mice. These data demonstrate the surprising effectiveness of the present invention as an antitumor and/or anti-metastatic therapeutic for inhibiting tumor growth and/or metastasis.

In summary, siDNA ODNs used against telomerase RNA template effectively inhibit tumor growth *in vitro* and *in vivo*. Treatment with ODNs is specific for telomerase RNA, and shows either comparable or better Telomerase RNA inactivation than conventional singlestranded antisense oligonucleotides, demonstrating an unexpected advantage over previous telomerase-based strategies.

### DETAILED DESCRIPTION AND PREFFERED EMBODIMENTS OF THE INVENTION

Under the term "homology" the following should be understood: An siDNA oligonucleotide according to the invention is consisting of an antisense-strand which is more than 80% homologous to the viral RNA target and a second strand, preferentially more than 90% homologous, which is partially complementary to the antisense-strand forming a partially double stranded hairpin-loop-structure. The term "partially complementary" means a homology between 40 and 60% within the same siDNA molecule.

Preferred specific examples according to the invention are siDNA oligonucleotides comprising the sequences SEQ ID NO 1:
ODN-hTR (= SEQ ID NO 1):
   5'-AAACAGATTCCCTAAGAGAGTTGGGTTTTCCCTTCTCAGTTAGGGTTAGACAAA-3'

siDNA molecules according to SEQ ID NO 1 are **characterized in that** the sequence forms a partially double-stranded, hairpin-loop structured oligonucleotide as shown below:

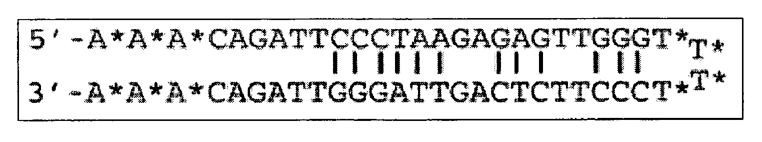

The asterisks indicate base modification of the phosphodiester backbone and vertical bars indicate Watson-Crick bonds.

In regards to the method for monitoring the RNA component of telomerase after treatment with siDNA oligonucleotides, comprising DNA oligonucleotides for the detection of the target RNA in order to determine the effect of the siDNA-mediated silencing, the following DNA oligonucleotides are preferred:
Forward Primer A ("hTR-F", SEQ ID NO 2):
   5'-GGTTGCGGAGGGTGGGTGGGC-3'
Reverse Primer B ("hTR-R", SEQ ID NO 3):
   5'-AACGGTGGAAGGCGGCA-3'

The invention is based on the fact that a DNA strand has a thermodynamic preference to form an RNA-DNA hybrid over double-stranded DNA or double-stranded RNA. Thus the invention is based on the unexpected result that siDNA is of high preference (in contrast to siRNA) to form RNA-DNA hybrids; siDNA is therefore of advantage and a preferred object of the invention. In contrast to siRNA, interferon-induction is a risk for siRNA and reduced or absent for an RNA-DNA hybrid or double-stranded DNA. Furthermore, siDNA is preferred due to the fact that DNA is easier to synthesize and more stable.

siDNA is superior to a simple antisense oligodeoxyribonucleotides, because it is more stable during uptake and inside the cell, and is therefore more effective. However, a siDNA-containing pharmaceutical agent can also contain ribonucleotides, siDNA/RNA chimeras, or can be combined with siRNAs.

Furthermore, siDNA oligonucleotides can be stabilized by base modifications. Such base modifications entail 2'-O-methylated nucleotides and phosphorothioates at the ends and in the central linker regions to protect against nucleases and to increase stability and longevity both in vivo and in pharmaceutical preparations. Other chemical modifications are also envisaged that may improve the stability of the siDNA oligonucleotides.

### MATERIALS AND METHODS USED IN THE FOLLOWING EXAMPLES

### Cell lines

The human cervical carcinoma cell line (Hela cells) and the murine metastatic melanoma cell line (B16-F10 cells) were purchased from American type culture collection (ATCC; Manassas, VA, USA). Both cell lines were cultured in Dulbecco's modified Eagle's medium (DMEM; Invitrogen, Carlsbad, CA, USA) supplemented with 10% heat-inactivation fetal bovine serum (Brunschwig AG, Basel, Switzerland) in the presence of 5% CO2 at 37°C.

### siDNA Oligonucleotides

ODN-hTR and ODN-mTR were designed for targeting the human and murine telomerase RNA template sequences, respectively. Briefly, ODNs were designed as hair-pinlooped structures where the antisense strand was fully complementary to the target site and linked by four additional Thymidines (T4 linker) to the second strand. All ODNs used were phosphorothioated at each end (three bases) and in the T4 linker. Antisense oligonucleotides (as-hTR and as-mTR) were designed complementary to their respective target site and were phosphorothioated at each end (three bases). All ODNs and antisense oligonucleotides were synthesized by Integrated DNA Technologies, Coralville, IA, USA. The siRNAs (si-hTR and si-mTR) were supplemented by dinucleotides dTdT overhangs at the 3' end of each strand (Dhar-macon, Lafayette, CO, USA). A non-specific scrambled ODN (ODN-sc) and an irrelevant siRNA (si-neg, Allstar siRNA; QIAGEN, Hilden, Germany) were used as negative controls.

### Transfection

Hela cells were seeded in the 12-well plate at the density of 16 x 104 cells/ml overnight. The following day cells were transfected with ODN-hTR, as-hTR, sihTR, ODN-sc or si-neg. Each oligonucleotide was diluted with 50ul of OPTIMMEM (Invitrogen). In a separate tube, 8ul of Lullaby (OZ Biosciences, Marseille Cedex, France) was diluted with OPTIM-MEM and the contents of the first tube were then added drop by drop into this tube. The mixture was incubated at room temperature for 20 min. This complex was then gently overlaid on culture cells in each well and mixed by gentle rocking for 30 sec. Mock-treated cells were transfected with lullaby alone. The first time of treatment was defined as 0 hour (0 h). Both cell lines were transfected with 50nM of each oligonucleotide for 16, 24 and 48 h.
B16-F10 cells were transfected as described above for Hela cells, except that they were transfected with mTR targeting oligonucleotides; ODN-mTR, asmTR, si-mTR.

### RNA preparation and quantitative Reverse Transcription Polymerase Chain Reaction (qRT-PCR)

Cellular RNA was isolated using QIAamp RNA Blood Mini kit (QIAGEN). Subsequent cDNA synthesis and qRT-PCR were performed in one step using the Quan-tiTect probe RT-PCR kit (QIAGEN) as recommended by manufacturer. The primers and Taqman probes were as follows: the hTR was amplified using forward primer hTR-F (5'-GGTTGCGGAGGGTGGGC-3', SEQ ID NO 2), reverse primer hTRR (5'-AACGGTGGAAGGCGGCAG-3', SEQ ID NO 3) and probe hTR-P (FAM-5'-TAGGCGCCGTGCTTTTGCTC-3'-TAMRA, SEQ ID NO 4). The human glyceraldehyde-3-phosphate dehydrogenase (hGAPDH) was used for normalization and was amplified by using forward primer hGAPDH-F (5'-GAAGGTGAAGGTCGGAGTC-3', SEQ ID NO 5), reverse primer hGAPDH-R (5'-GAAGATGGTGATGGGATTTC-3', SEQ ID NO 6) and probe hGAPDH-P (FAM-5'-CAAGCTTCCCGTTCTCAGCC-3'-TAMRA, SEQ ID NO 7). The mTR was amplified using forward primer mTR-F (5'-GTCTTTTGTTCTCCGCCCG-3', SEQ ID NO 8), reverse primer mTR-R (5'-CGGCGAACCTGGAGCTC-3', SEQ ID NO 9) and probe mTR-P (FAM-5'-CGTTCCCGAGCCTCAAAAACAAACG-3'-TAMRA, SEQ ID NO 10). The mouse GAPDH (mGAPDH) was amplified by using forward primer mGAPDH-F (5'-CTTCACCACCATGGAGAAGGC-3', SEQ ID NO 11), reverse primer mGAPDH-R (5'-GGCATGGACTGTGGTCATGAG-3', SEQ ID NO 12 ) and probe mGAPDH-P (FAM-5'- CCTGGCCAAGGTCATCCATGACAACTTT -3'-TAMRA, SEQ ID NO 13). Cycling conditions were used as: 48°C for 30 min (1 cycle for cDNA synthesis), 95°C for 10 min (1 cycle) and 95°C for 15 sec followed by 60°C (69°C for hTR) for 1 min (50 cycles). GAPDH was used for standardization. Percentage RNA values were compared to mock-treated cells. Bars represent means of relative hTR RNA level ± standard deviation (±SD) from three independent experiments.

### Cell proliferation and viability

The effect of ODNs on cell proliferation was determined by cell proliferation kit I (Roche Diagnostics GmbH, Mannheim, Germany) using [4, 5-dimethylthiazolzyl]-2, 5-diphenyltetrazolium bromide (MTT) reagent. Cells were seeded in triplicates in 12-well plate with the density of 8 x 104 cells/ml. On following day at 0 h, cells transfected with oligonucleotides as described above. After 48 h of transfection, cells were trypsinized and 2 x 103 cells in 100µl from each well were transferred into 96-well plate and incubated for overnight. The MTT assay was performed at 72 has recommended by manufacturer. Absorbance was measured at 550nm/650nm to determine the cell viability and was compared to mock-treated cells to obtain relative cell proliferation. Bar represents relative cell proliferation ±SD from two independent experiments.

### Apoptosis assay

Apoptotic Hela cells were detected using M30-Apoptosense ELISA kit (PEVIVA AB, Bromma, Sweden) which measures the level of soluble cytokeratin 18 (CK 18). Cells were seeded in triplicates in 12-well plate with the density of 8 x 104 cells/ml and incubated for 24 h at 37°C. At 0 h, cells were transfected using a procedure as described above for Hela cells. After 72h of transfection, apoptotic assay was performed as recommended by manufacturer and absorbance was measured at 450nm. Bars represent the absorbance at 450nm ±SD from two independent experiments. Positive control represents the M30-high control as provided in the kit.

### Overexpression of p66 subunit of HIV-1 RT/RNase H in Hela cells

The catalytic active p66 subunit of HIV-1 RT/RNase H expressed in codonoptimized 96ZM651.8 clone (AIDS Reagents Cat# 6173) was cloned into pIRES-NEOHA/FLAG. Hela cells were pre-transfected (-48 h) with 6µg of the p66 or control plasmid in 10-cm plate. After 24 h, cells were trypsinized and seeded for overnight in 12-well plate. Cells were transfected with oligonucleotides at 0 h using the procedure as described above for Hela cells. After 48 h of transfection, the amount of hTR RNA was checked by qRT-PCR. Relative RNA values were obtained by comparison to mock-treated cells. Bars represent relative hTR RNA level ±SD from two independent experiments.

### Knockdown of cellular RNase H1 and RNase H2A in Hela cells

RNase H1- and RNase H2A-deficient Hela cells were generated by pretransfection (-48 h) of cells in 10-cm plate with 50nM of RNases H-specific siRNAs (QIAGEN, except where stated) or control irrelevant siRNA (si-neg). A small d within the sequence marks the corresponding base as a deoxyribonucleotide. The siRNAs for knocking down RNase H1 were; 1): siRnH1-1s (sense): 5'-GUUUGCCACAGAGGAUGAGdTdT-3' (SEQ ID NO 14), siRnH1-1as (antisense): 5'-CUCAUCCUCUGUGGCAAACdTdG-3' (SEQ ID NO 15, synthesized by Dhar-macon); 2) siRnH1-2s (sense): 5'-CGAUAAAUGGUAUAACUAAdTdT-3' (SEQ ID NO 16), siRnH1-2as (antisense) :5'-UUAGUUAUACCAUUUAUCGdTdA-3' (SEQ ID NO 17); 3) siRnH1-3s (sense) :5'-GGUUAAGUAUAUAAUAAAUdTdT-3' (SEQ ID NO 18), siRnH1-3as (antisense) : 5'-AUUUAUUAUAUACUUAAACCdAdT-3' (SEQ ID NO 19). The siRNA for knocking down RNase H2A were; 1): siRnH2A-1s (sense): 5'-GGACUUGGAUACUGAUUAUdTdT-3' (SEQ ID NO 20), siRnH2A-1as (antisense): 5'-AUAAUCAGUAUCCAAGUCCdTdG-3' (SEQ ID NO 21); 2) siRnH2A-2s (sense): 5'-GGAUUGAGGUGACGGUCAAdTdT-3' (SEQ ID NO 22), siRnH2A-2as (antisense): 5'-UUCAGGUUGUAUUUGACCCdGdC-3' (SEQ ID NO 23); 3) siRnH2A-3s (sense): 5'-GGGUCAAAUACAACCUGAAdTdT-3' (SEQ ID NO 24), siRnH2A-3as (antisense): 5'-UUCAGGUUGUAUUUGACCCdGdC-3' (SEQ ID NO 25). After 24 h of pre-transfection, cells were trypsinized and 16 x 104 cells/ml were plated into 12-well plate. Next day, cells were transfected with ODN-hTR, as-hTR, si-hTR, ODN-sc or si-neg using the procedure as described above for Hela cells. The time of transfection was defined as 0 h. After 48 h of transfection, the amount of hTR RNA was checked by qRT-PCR. Relative RNA values were obtained by comparison to mock-treated cells. Bars represent relative hTR RNA level ±SD from two independent experiments.

### In vivo studies

C57BL/6 mice were bred in the animal facility of the Institute of Medical Virology and used at 6-8 weeks of age. The B16-F10 cells were transfected with 50nM of ODN-mTR, as-mTR, si-mTR, ODN-sc, si-neg or mock-treated cells as described above. For comparison untransfected control cells were used. The time of treatment was defined as 0 h. After 16 h of transfection at 37°C, cells were trypsinized and washed twice with PBS. Numbers of cells were counted and adjusted to 2 x 105 cells in a total volume of 200µl. The pulmonary metastases formation was established by intravenous injection (i.v.) of transfected or untransfected control cells into the tail vein of C57BL/6 mice. Twenty one days (d 21) later the animals were sacrificed and numbers of metastases were examined by visual inspection of lungs (Schultz et al., 1999). The results are shown as numbers of metastases in individual mice. Bars represent relative numbers of metastases ±SD compared to control cells from two independent experiments.

**Table 1: Oligonucleotides of the present invention. All sequences are listed in 5'-3' orientation. A small d within the RNA sequences marks the corresponding base as a deoxyribonucleotide.**

| **SEQ ID NO** | **SEQUENCE (5'-3')** | **OTHER NAME** | **DNA/ RNA** |
|---|---|---|---|
| 1 | AAACAGATTCCCTAAGAGAGTTGGGTTTTCCCTTC- TCAGTTAGGGTTAGACAAA | ODN-hTR | DNA |
| 2 | GGTTGCGGAGGGTGGGTGGGC | hTR -F | DNA |
| 3 | AACGGTGGAAGGCGGCA | hTR -R | DNA |
| 4 | TAGGCGCCGTGCTTTTGCTC | hTR -P | DNA |
| 5 | GAAGGTGAAGGTCGGAGTC | hGAPDH-F | DNA |
| 6 | GAAGATGGTGATGGGATTTC | hGAPDH-R | DNA |
| 7 | CAAGCTTCCCGTTCTCAGCC | hGAPDH-P | DNA |
| 8 | GTCTTTTGTTCTCCGCCCG | mTR -F | DNA |
| 9 | CGGCGAACCTGGAGCTC | mTR -R | DNA |
| 10 | CGTTCCCGAGCCTCAAAAACAAACG | mTR -P | DNA |
| 11 | CTTCACCACCATGGAGAAGGC | mGAPDH-F | DNA |
| 12 | GGCATGGACTGTGGTCATGAG | mGAPDH-R | DNA |
| 13 | CCTGGCCAAGGTCATCCATGACAACTTT | mGAPDH-P | DNA |
| 14 | GUUUGCCACAGAGGAUGAGdTdT | siRnH1-1s | RNA |
| 15 | CUCAUCCUCUGUGGCAAACdTdG | siRnH1-1as | RNA |
| 16 | CGAUAAAUGGUAUAACUAAdTdT | siRnH1-2s | RNA |
| 17 | UUAGUUAUACCAUUUAUCGdTdA | siRnH1-2as | RNA |
| 18 | GGUUAAGUAUAUAAUAAAUdTdT | siRnH1-3s | RNA |
| 19 | AUUUAUUAUAUACUUAAACCdAdT | siRnH1-3as | RNA |
| 20 | GGACUUGGAUACUGAUUAUdTdT | siRnH2A-1 s | RNA |
| 21 | AUAAUCAGUAUCCAAGUCCdTdG | siRnH2A-1as | RNA |
| 22 | GGAUUGAGGUGACGGUCAAdTdT | siRnH2A-2s | RNA |
| 23 | UUCAGGUUGUAUUUGACCCdGdC | siRnH2A-2as | RNA |
| 24 | GGGUCAAAUACAACCUGAAdTdT | siRnH2A-3s | RNA |
| 25 | UUCAGGUUGUAUUUGACCCdGdC | siRnH2A-3as | RNA |
| 26 | CCCTTCTCAGTTAGGGTTAGACAAA | as-hTR | DNA |
| 27 | UUGUCUAACCCUAACUGAGdTdT | si-hTR | RNA |
| 28 | TTGTTTCCTGGAGAGTTGGACCAGGTTTTCCTCC- TCCTTCTCTCCTCCTTTATG | ODN-sc | DNA |
| 29 | TGGATTGGGTGATTTAGATTTCCTGTTTTCAGC- | ODN-mTR | DNA |
| | TAATGAAAATCAGGGTTAGGT | | |
| 30 | CAGCTAATGAAAATCAGGGTTAGGT | as-mTR | DNA |
| 31 | ACCUAACCCUGAUUUUCAUdTdT | si-mTR | RNA |

### EXAMPLES

### 1. Effect of ODNs on telomerase RNA template

The RNA component of telomerase is essential for telomerase reverse transcription and is therefore a natural target for anti-telomerase agents (Figure 1a). Several groups have described the ability of antisense oligonucleotides and siRNAs to target the hTR. A new class of oligodeoxynucleotide, partially doublestranded DNA, ODN, has been successfully used in targeting HIV-1 RNA. Thus, we tested for ODN-mediated hTR inhibition in comparison with its antisense and siRNA analogues. A set of oligonucleotides; ODN-hTR (SEQ ID NO 1), as-hTR (SEQ ID NO 26), si-hTR (SEQ ID NO 27) targeting hTR was designed (Figure 1b). ODN-sc was used as a control (SEQ ID NO 28). To evaluate the onset and duration of action of each oligonucleotide, Hela cells were transfected with 50nM of each oligonucleotide and the hTR RNA level was measured at 16, 24 and 48 h of transfection using qRT-PCR. Minimal decrease in the hTR RNA was observed after 16 and 24 h of transfection. However, at 48 h the level of hTR RNA was strongly reduced to 32 ± 8 by ODN-hTR, 48 ± 4 by as-hTR and 36 ± 8 % (SD) by si-hTR relative to the mock-treated cells (Figure 1c). Further decrease or increase in concentration did not improve hTR knockdown level (data not shown).

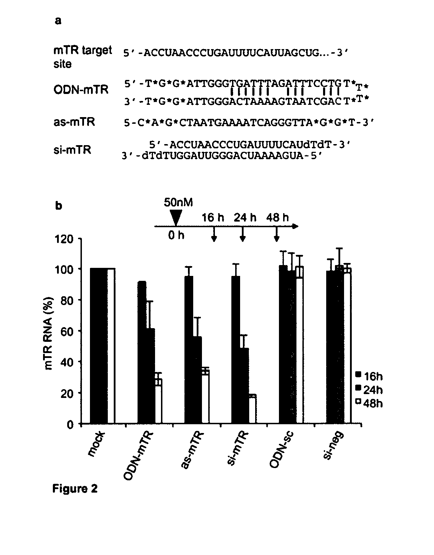

In order to facilitate the functional utility of ODN in a mouse model in vivo, we used ODN to target the mouse telomerase as well. The ability of siRNA targeting the murine TERT (mTERT) had been described previously, but no report has been published for targeting the mouse TR (mTR). Here, we designed a set of oligonucleotides; ODN-mTR (SEQ ID NO 29), as-mTR (SEQ ID NO 30), si-mTR (SEQ ID NO 31) targeting mTR (Figure 2a) and first tested their efficiency and specificity in vitro using B16-F10 cells. The cells were tansfected with 50nM of each oligonucleotide and expression level of the mTR was measured at 16, 24 and 48 h of transfection using qRT-PCR. At 48 h, the level of mTR RNA was strongly reduced to 29 ± 4 by ODN-mTR, 34 ± 2 by as-mTR and 18 ± 1 % (SD) by simTR relative to mock-treated cells (Figure 2b).

Since oligonucleotides can cause non-sequence-specific effects, control ODN-sc and si-neg were used. No effect on RNA level was observed in both cell lines, suggesting sequence specificity of the hTR and mTR targeting oligonucleotides.

Under our transfection conditions, significant inhibition of gene expression was observed in respective cell lines and this effect was highly sequence specific. In Hela cells, the effect of ODN-hTR to inhibit the hTR RNA was higher than its analogous single-stranded antisense oligonucleotide (ashTR) and equal to siRNA (si-hTR) (Figure 1c). In B16-F10 cells, the ODN-mTR inhibitory effect on the mTR RNA was also higher than its analogue singlestranded antisense oligonucleotide (as-mTR) but lower than siRNA (si-mTR) (Figure 2b).

### 2. Cell proliferation assay

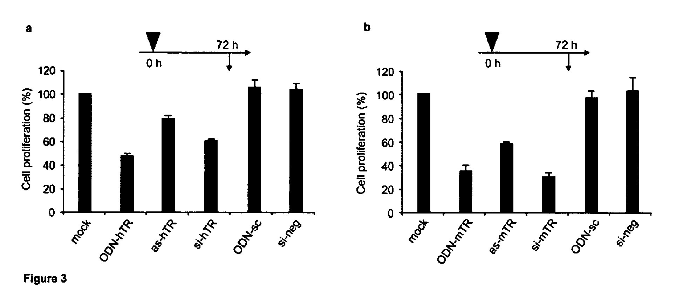

Using phase-contrast microscopy, we noticed that oligonucleotides had variable effect on cell proliferation during telomerase RNA knockdown studies (data not shown). In order to quantify the potential effect of these oligonucleotides on cell proliferation, an MTT assay was performed after 72 h of oligonucleotides transfection. This has been used by others as a mean of assaying the quantification of viable cells, because only metabolic active cells cleave the MTT salt to form formazan dye. Absorbance was measured at 550nm/650nm to determine the cell viability and was compared to mock-treated cells to obtain relative cell proliferation. In Hela cells, the relative cell proliferation was 48 ± 1.9 by ODN-hT, 80 ± 2.1 by ashTR and 61 ± 1.69 % (SD) by si-hTR (Figure 3a). In B16-F10 cells, relative cell proliferation was 35 ± 5 by ODN-mT, 58 ± 1 by as-mTR and 30 ± 3 % (SD) by simTR (Figure 3b). No effect on cell proliferation was observed by ODN-sc and sineg in both cell lines. We conclude that reduction in cell proliferation was specific to the inhibition of hTR or mTR level in Hela or B16-F10 cells, respectively.

Reduction in cell proliferation was detectable rather after short period (72 h) of oligonucleotides transfection. The effect of ODN-hTR and -mTR on cell proliferation inhibition was higher compared to the antisense oligonucleotide. However, this effect was either higher or equal to its siRNA analogue, depending on targeting either the hTR or mTR.

### 3. Apoptosis

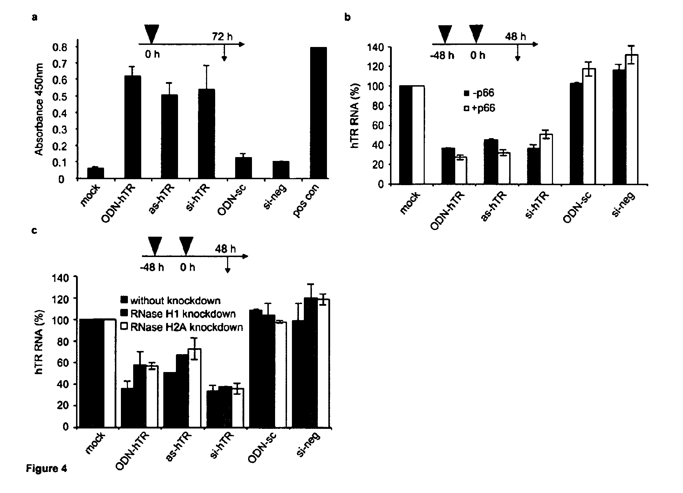

As inhibition of hTR in vitro is known to limit cell life span by triggering cell apoptosis, we tested whether ODN-mediated hTR downregulation was correlated with apoptosis. Hela cells were transfected with oligonucleotides at 0 h and apoptosis ELISA assay was performed after 72 h of transfection. The absorbance was measured at 450nm which is directly proportional to the concentration of apoptotic-associated CK18 in cell lysates. The absorbance was increased to 0.62nm by ODN-hTR, 0.51 nm by as-hTR and 0.53nm by si-hTR compared to 0.063nm by mock-treated cells (Figure 4a). This data indicates that the apoptotic cell death was responsible for rapid inhibition of Hela cell proliferation. The effect of ODN-hTR on the induction of apoptosis was again higher than as-hTR and equal to si-hTR. Induction of apoptosis by all three tested oligonucleotides was stronger when compared to mock-treated cells.

### 4. Role of RNase H

The ability of antisense oligonucleotides to elicit RNase H degradation of target mRNA is one of the most important criteria for antisense effectiveness. Here, we speculated that ODN-hTR may generate RNA-DNA hybrids while targeting hTR, if so, then cellular RNase H might have a role in ODN-mediated hTR downregulation in Hela cells. In order to test this mechanism, the catalytically active p66 subunit of HIV-1-RT/RNase H or control plasmid was transiently overexpresed in Hela cells. At 0 h, cells were transfected with hTR specific or control oligonucleotides for 48 h. The hTR RNA level was decreased from 36.5 ± 0.7 to 27.5 ± 2 % (SD) by ODN-hTR and from 45.5 ± 0.7 to 32 ± 2.8 % by as-hTR in the p66 overexpressing (+p66) cells compared to control (-p66). In contrast, no further decrease was observed by si-hTR transfection.

To test this further, cellular RNases H were knocked down in Hela cells. In the first step, a control (without knockdown) study was performed. Hela cells were pre-transfected (-48 h) with control irrelevant siRNA (si-neg) and later, at 0 h, transfected with the hTR specific oligonucleotides. The hTR RNA level was reduced to 36 ± 8 by ODN-hTR, 51 ± 0.29 by as-hTR and 34 ± 5 % (SD) by sihTR relative to mock-treated cells. In the next step, cells were pre-transfected (-48 h) with siRNAs specific to RNase H1 (RNase H1 knockdown) or RNase H2A (RNase H2A knockdown). The knocked down effect of these siRNAs was checked by qRT-PCR using primers specific for RNase H1 and RNase H2A, respectively (data not shown). Later, at 0 h, these cells were transfected with the hTR specific oligonucleotides. In RNase H1 knocked down cells, the hTR RNA level was reduced to 58 ± 13 by ODN-hTR, 67 ± 0.16 by as-hTR and 38 ± 0.15 % (SD) by si-hTR compared to mock-treated cells. In RNase H2A knocked down cells, hTR RNA level was reduced to 57 ± 3 by ODN-hTR, 73 ± 10 by as-hTR and 36 ± 5 (SD) by si-hTR compared to mock-treated cells. These results showed a decrease in inhibition of hTR by ODN- and as-hTR in knocked down cells compared to control (without knockdown). Thus, both cellular RNases H appear to contribute to the anti-proliferative effect of ODN.

We have investigated the role of RNase H by transient expression of the p66 subunit of HIV-1 RT/RNase H and by knocking down of RNase H1 or RNase H2A in Hela cells. Over-expressing p66 enhanced the inhibition of hTR by ODN- and as-hTR (Figure 4b), whereas knocking down both RNases H led to a decrease in hTR RNA inhibition by these oligonucleotides (Figure 4c). These results suggest a role of these enzymes in ODN-mediated inhibition of the hTR. This effect was sequence specific as no relevant effect was observed by si-hTR and controls.

### 5. Suppression of lung metastasis in mice by intravenous injection of the mTR transfected B16-F10 melanoma cells

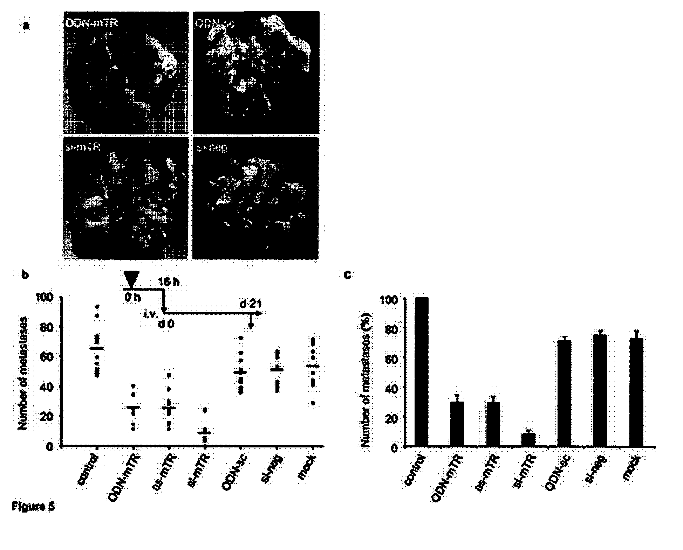

As oligonucleotides targeting the mTR in B16-F10 cells inhibited both the mTR RNA level and cell proliferation in vitro, we explored their activity on in vivo tumor growth. The animal model used here was the well-described B16-F10/C57BL/6 system for metastatic malignant melanoma. After 16 h of 10 oligonucleotides transfection, B16-F10 cells were injected into tail vein of C57BL/6 mice. The metastases observed in the lungs were pigmented by melanin and were easily detectable on opening the chest of mice after 21 days (Figure 5a). Number of metastases were counted and found to be decreased to 26 ± 3 by ODN-mTR, 25 ± 4 by as-mTR and 8.6 ± 3 (SD) by si-mTR (Figure 5b) compared to 65 ± 5 (SD) by control untransfected cells. Injection of ODN-sc or sineg-transfected B16-F10 cells had no significant effect on metastases formation. Results from two independent experiments showed a reduction in the numbers of metastases to 30 % by ODN-mTR, 29.5 % by as-mTR and 8.5 % by si-mTR when compared to control (Figure 5c).

Significant reduction in numbers of lung metastases was found after i.v. administration of ODN-mTR-transfected B16-F10 cells compared to control untransfected cells (Figure 5). Similarly as- or si-mTR-transfected B16-F10 cells also showed the reduced numbers of metastases, whereby si-mTR showed stronger effect.

### DETAILED DESCRIPTION OF THE FIGURES

**Figure 1**. Oligonucleotides targeting the human telomerase RNA (hTR). (a) Model on telomeric DNA synthesis catalysed by human telomerase. Human telomerase catalytic protein (hTERT) is shown with the template sequence spaning nucleotides +46 to +56 of the hTR. The 3' end of the telomeric DNA base pairs with the template region of the telomerase RNA and extend the sequence until the 5' end of template is reached. After this, the telomeric DNA undergoes translocation and re-alignment with the 3' end of template for further round of extension. Telomeres repeat sequence is shown in bold letters. (b) The sequences of the hTR (target site) and hTR targeting ODN (ODN-hTR), antisense oligonucleotide (as-hTR) and siRNA (si-hTR) are shown. For control, the sequence of non-specific scrambled ODN (ODN-sc) is shown. Phosphorothioation of the phosphodiester backbone is indicated by asterisks. Vertical bars indicate Watson-Crick bonds. (c) Hela cells (16 x 104 cells/ml) were transfected at 0 h with the indicated oligonucleotides (50nM). RNA was extracted at various time points 16, 24 and 48 h and analyzed by qRT-PCR using a set of primers recognizing the template region of hTR. Human GAPDH (hGAPDH) was used for standardization. Bars represent mean values of hTR RNA (±SD) from three different experiments, compared to mock-treated cells. Sequence of events is summarized by horizontal time line in which time of oligonucleotides transfection is indicated as triangle and RNA extraction as downward arrow.
**Figure 2****.** Oligonucleotides targeting murine telomerase RNA (mTR). (a) The murine equivalent sequences of the mTR (target site) and mTR targeting ODN (ODN-mTR), antisense oligonucleotide (as-mTR) and siRNA (si-mTR) are shown. (b) B16-F10 cells (16 x 104 cells/ml) were transfected at 0 h with the indicated oligonucleotides (50nM). RNA was extracted at various time points 16, 24 and 48 h and analyzed by qRT-PCR using a set of primers recognizing the template region of mTR. Murine GAPDH (mGAPDH) was used for standardization. Bars represent mean values of mTR RNA (±SD) from three different experiments, compared to mock-treated cells. Sequence of events is summarized by horizontal time line in which time of oligonucleotides transfection is indicated by triangle and RNA extraction by downward arrow.
**Figure 3****.** In vitro analysis of tumor cell proliferation. (a) Hela cells (8 x 10⁴ cells/ml) were transfected at 0 h with the indicated oligonucleotides (50nM). MTT assay was performed at 72 h of transfection as described in Materials and Methods. Absorbance was measured at 550nm/650nm to determine the cell viability and was compared to mock-treated cells to obtain relative cell proliferation. Bars represent relative cell proliferation ±SD from two independent experiments. Sequence of events is summarized by horizontal time line in which time of oligonucleotides transfection is indicated by triangle and MTT assay by downward arrow. (b) Same as a except that murine B16-F10 cells (8 x 104 cells/ml) were transfected with the indicated oligonucleotides (50nM).
**Figure 4****.** Mechanism of ODN-mediated hTR downregulation. (a) Quantification of cell apoptosis. Hela cells (8 x 104 cells/ml) were transfected at 0 h with the indicated oligonucleotides. Apoptosis ELISA assay was performed at 72 h of transfection as described in Material and Methods. Absorbance was measured at 450nm. Bars represent the absorbance at 450nm ±SD from two independent experiments. Positive control (pos con) represents the M30-high control as provided in the kit. Sequence of events is summarized by horizontal time line in which time of oligonucleotides transfection is indicated by triangle and apoptosis ELISA assay by downward arrow. (b) Hela cells were pre-transfected (-48 h) with control (-p66) or with the p66 plasmid (+p66) and at 0 h transfected with the indicated oligonucleotides. RNA was extracted at 48 h and analyzed by qRTPCR using a set of primers recognizing the template region of hTR. Human GAPDH (hGAPDH) was used for standardization. Relative RNA values were obtained by comparison to mock-treated cells. Bars represent relative hTR RNA ±SD from two independent experiments. Sequence of events is summarized by horizontal time line in which time of oligonucleotides transfection is indicated as triangle and RNA extraction as downward arrow. (c) Similar as b except that Hela cells were pre-transfected with control irrelevant siRNA (without knockdown) or with siRNAs specific for RNase H1 (RNase H1 knockdown) and RNase H2A (RNase H2A knockdown).
**Figure 5**. In vivo anti-metastatic effect. (a) Lung metastases after intravenous (i.v.) administration of transfected B16-F10 cells with ODN-mTR verses ODN-sc and si-mTR verses si-neg are shown. The globular dark structures indicate the metastases (right top and bottom). (b) B16-F10 cells (16 x 104 cells/ml) were transfected at 0 h with the indicated oligonucleotides (50nM). After 16 h, cells were trypsinized and numbers of cells were adjusted to 2 x 105 cells in a total volume of 200µl. The pulmonary metastases formation was established by i.v. administration of transfected or untransfected control cells into tail vein of C57BU6 mice at day 0 (d 0). The animals were sacrificed at day 21 (d 21) and numbers of metastases were examined by visual inspection of lungs. Numbers of metastases in individual mice are indicated by dots and the average by dash. Sequence of events is summarized by horizontal time line in which time of oligonucleotides transfection is indicated as triangle, i.v. administration at d 0 and time to sacrifice the mice at d 21 by downward arrows. (c) Total number of metastases from two independent experiments with 5-10 mice per/group. Bars represent relative numbers of metastases ±SD compared to untransfected control.

In regards to the sequence protocol, despite having been correctly entered as DNA/RNA hybrid molecules, sequences 14-25, 27 and 31 are displayed in the printed form under code <212> as DNA sequences. This appears to be a software error. These sequences represent RNA oligos, wherein the last two bases are DNA nucleotides.

The molecules are correctly described in the description (see Table 1).

## Claims

1. Isolated and purified siDNA oligonucleotides for binding to one or more RNA target regions of the RNA component of telomerase, comprising a partially double-stranded, hairpin-loop structure with an antisense-strand complementary to one or more of said RNA target regions and a second strand, partially complementary to the antisense strand, wherein the siDNA oligonucleotides bind to one or more target regions of the RNA component of telomerase and serve as an antitumor and/or anti-metastatic therapeutic for inhibiting tumor growth and/or metastasis.

2. The isolated and purified siDNA oligonucleotides according to claim 1, wherein the siDNA oligonucleotide is complementary to the template region of the RNA component of telomerase of humans.

3. The isolated and purified siDNA oligonucleotides according to claim 1, wherein the siDNA molecule is complementary to one or more conserved regions of the RNA component of telomerase, of at least 20 nucleotides in length, wherein the antisense strand of siDNA is more than 80%, more preferably more than 90% homologous to the target telomerase RNA.

4. The isolated and purified siDNA oligonucleotides according to claim 1, whereby the second strand is connected to the antisense strand through a thymidine linker, preferred 4 nucleotides in length, where the second strand has a homology of 40 to 60% to the antisense-strand and is partially complementary to the antisense-strand.

5. The isolated and purified siDNA oligonucleotides according to claim 1, wherein the siDNA oligonucleotide is stabilized by base modifications.

6. The isolated and purified siDNA oligonucleotides according to claim 1, comprising the sequence SEQ ID NO 1.

7. The isolated and purified siDNA oligonucleotides according to claim 6, **characterized in that** the sequence is forming a partially double-stranded, hairpin-loop structured oligonucleotide as shown below 
5'-A*A*A*CAGATTCCCTAAGAGAGTTGGGT*T*
||||||| ||| | |||
3'-A*A*A*CAGATTGGGATTGACTCTTCCCT*T*
 whereby asterisks indicate base modification of the phosphodiester backbone and vertical bars indicate Watson-Crick bonds.

8. Method for monitoring the RNA component of telomerase after treatment with siDNA oligonucleotides according to claim 1, comprising DNA oligonucleotides for the detection of the target RNA in order to determine the effect of the siDNA-mediated silencing, wherein the following DNA oligonucleotides are used:
| | |
|---|---|
| Forward Primer A ("hTR-F"): | 3'-CGGGTGGGTGGGAGGCGTTGG-5' |
| Reverse Primer B ("hTR-R"): | 3'-ACGGCGGAAGGTGGCAA-5' |

9. Method for the treatment of cancer and/or tumor growth comprising: administering siDNA, or combination of two or three siDNAs according to claim 1, wherein the siDNA oligonucleotides bind to one or more target regions of the RNA component of telomerase and serve as an antitumor and/or anti-metastatic therapeutic for inhibiting tumor growth and/or metastasis.

10. Pharmaceutical composition as an antitumor and/or anti-metastatic therapeutic for inhibiting tumor growth and/or metastasis comprising siDNA oligonucleotides according to claim 1, wherein the siDNA oligonucleotides are capable of binding to one or more target regions of the RNA component of telomerase.

11. Pharmaceutical composition according to claim 10 for the treatment of cancer, in particular malignant lung metastases.


SEQUENCE LISTING

<110> Mölling, Karin

<120> Anti-tumor activity of small double-stranded
oligodeoxynucleotides targeting telomerase RNA

<130> 665/09

<150> EP08075678
<151> 2008-07-31

<160> 31

<170> PatentIn version 3.3

<210> 1
<211> 54
<212> DNA
<213> Synthetic

<220>
<221> modified_base
<222> (1)..(3)

<220>
<221> modified_base
<222> (52)..(54)

<400> 1
aaacagattc cctaagagag ttgggttttc ccttctcagt tagggttaga caaa 54

<210> 2
<211> 21
<212> DNA
<213> Synthetic

<400> 2
ggttgcggag ggtgggtggg c 21

<210> 3
<211> 17
<212> DNA
<213> Synthetic

<400> 3
aacggtggaa ggcggca 17

<210> 4
<211> 20
<212> DNA
<213> Synthetic

<400> 4
taggcgccgt gcttttgctc 20

<210> 5
<211> 19
<212> DNA
<213> Synthetic

<400> 5
gaaggtgaag gtcggagtc 19

<210>  6
<211>  20
<212>  DNA
<213>  Synthetic

<400>  6
gaagatggtg atgggatttc                                                    20


<210>  7
<211>  20
<212>  DNA
<213>  Synthetic

<400>  7
caagcttccc gttctcagcc                                                    20


<210>  8
<211>  19
<212>  DNA
<213>  Synthetic

<400>  8
gtcttttgtt ctccgcccg                                                     19


<210>  9
<211>  17
<212>  DNA
<213>  Synthetic

<400>  9
cggcgaacct ggagctc                                                       17


<210>  10
<211>  25
<212>  DNA
<213>  Synthetic

<400>  10
cgttcccgag cctcaaaaac aaacg                                              25


<210>  11
<211>  21
<212>  DNA
<213>  Synthetic

<400>  11
cttcaccacc atggagaagg c                                                  21


<210>  12
<211>  21
<212>  DNA
<213>  Synthetic

<400>  12
ggcatggact gtggtcatga g                                                  21


<210>  13
<211>  28
<212>  DNA

<213>  Synthetic

<400>  13
cctggccaag gtcatccatg acaacttt                                          28


<210>  14
<211>  21
<212>  DNA
<213>  Synthetic


<220>
<221>  DNA
<222>  (20)..(21)

<400>  14
guuugccaca gaggaugagt t                                                 21


<210>  15
<211>  21
<212>  DNA
<213>  Synthetic


<220>
<221>  DNA
<222>  (20)..(21)

<400>  15
cucauccucu guggcaaact t                                                 21


<210>  16
<211>  21
<212>  DNA
<213>  Synthetic


<220>
<221>  DNA
<222>  (20)..(21)

<400>  16
cgauaaaugg uauaacuaat t                                                 21


<210>  17
<211>  21
<212>  DNA
<213>  Synthetic


<220>
<221>  DNA
<222>  (20)..(21)

<400>  17
uuaguuauac cauuuaucgt a                                                 21


<210>  18
<211>  21
<212>  DNA
<213>  Synthetic

<220>
<221> DNA
<222> (20)..(21)

<400> 18
gguuaaguau auaauaaaut t 21


<210> 19
<211> 22
<212> DNA
<213> Synthetic


<220>
<221> DNA
<222> (21)..(22)

<400> 19
auuuauuaua uacuuaaacc at 22


<210> 20
<211> 21
<212> DNA
<213> Synthetic


<220>
<221> DNA
<222> (20)..(21)

<400> 20
ggacuuggau acugauuaut t 21


<210> 21
<211> 21
<212> DNA
<213> Synthetic


<220>
<221> DNA
<222> (20)..(21)

<400> 21
auaaucagua uccaagucct g 21


<210> 22
<211> 21
<212> DNA
<213> Synthetic


<220>
<221> DNA
<222> (20)..(21)

<400> 22
ggauugaggu gacggucaat t 21


<210> 23
<211> 21
<212> DNA
<213> Synthetic

<220>
<221> DNA
<222> (20)..(21)

<400> 23
uucagguugu auuugacccg c                                                21


<210> 24
<211> 21
<212> DNA
<213> Synthetic


<220>
<221> DNA
<222> (20)..(21)

<400> 24
gggucaaaua caaccugaat t                                                21


<210> 25
<211> 21
<212> DNA
<213> Synthetic


<220>
<221> DNA
<222> (20)..(21)

<400> 25
uucagguugu auuugacccg c                                                21


<210> 26
<211> 25
<212> DNA
<213> Synthetic

<400> 26
cccttctcag ttagggttag acaaa                                            25


<210> 27
<211> 21
<212> DNA
<213> Synthetic


<220>
<221> DNA
<222> (20)..(21)

<400> 27
uugucuaacc cuaacugagt t                                                21


<210> 28
<211> 54
<212> DNA
<213> Synthetic


<220>

<221>  modified_base
<222>  (1)..(3)

<220>
<221>  modified_base
<222>  (52)..(54)

<400>  28
ttgtttcctg gagagttgga ccaggttttc ctcctccttc tctcctcctt tatg          54


<210>  29
<211>  54
<212>  DNA
<213>  Synthetic


<220>
<221>  modified_base
<222>  (1)..(3)

<220>
<221>  modified_base
<222>  (52)..(54)

<400>  29
tggattgggt gatttagatt tcctgttttc agctaatgaa aatcagggtt aggt          54


<210>  30
<211>  25
<212>  DNA
<213>  Synthetic

<400>  30
cagctaatga aaatcagggt taggt                                          25


<210>  31
<211>  21
<212>  DNA
<213>  Synthetic


<220>
<221>  DNA
<222>  (20)..(21)

<400>  31
accuaacccu gauuuucaut t                                              21